# EUROPEAN PATENT SPECIFICATION

(11) **EP 0 327 345 B1**
(45) Date of publication and mention of the grant of the patent: **20.10.1993**
(21) Application number: 89300985.2
(22) Date of filing: 01.02.1989
(51) Int. Cl.: A61K 7/13

(54) **Hair darkening compositions**
Präparate zum Dunklermachen der Haare
Composition pour rendre plus foncé les cheveux

(30) Priority: 04.02.1988 GB 8802455
(43) Date of publication of application: 09.08.1989
(73) Proprietor: Wella Aktiengesellschaft, 64274 Darmstadt (DE)
(72) Inventor: Wilkins, Anne Lilian Beecham Products, Weybridge Surrey KT13 0DE (GB); Day, Martin Edwin Beecham Products, Weybridge Surrey KT13 0DE (GB); Rowland, Mark Louis Beecham Products, Weybridge Surrey KT13 0DE (GB)

(56) References cited:
- EP-A- 0 173 178
- EP-A- 0 216 334
- EP-A- 0 328 816
- DE-A- 2 028 818
- GB-A- 2 173 515
- CHEMICAL ABSTRACTS, vol. 94, no. 14, April 1981, page 381, abstract no. 109084x, Columbus, Ohio, US; & JP-A-80 154 912 (SUNSTAR) 02-12-1980

## Description

The present invention relates to compositions which are useful for darkening natural fibres and, in particular, for darkening human hair, to a method of darkening such hair and to kits comprising the darkening composition.

Natural fibres such as human hair can be dyed in a variety of ways, depending on the required duration of the colouring. Temporary colouration is achieved using, as dyes, compounds of large molecular weight so that the molecules of the compound cannot penetrate the cortex of the hair. Semi-permanent colouration is effected using, as dyes, compounds of lower molecular weight, such as direct dyes, which have good affinity for hair keratin and are able to penetrate the cortex. Direct dyes, as the name implies, are applied directly to the hair, without the need for subsequent transformation. Permanent colouration is accomplished by the use of oxidation dyes. These are colourless intermediates which are transformed on the hair by a series of chemical reactions, involving, in general, oxidation (which is almost always effected by hydrogen peroxide), followed by coupling and further oxidation, to produce, in situ, the desired colour. Obviously such a process is complex and time consuming and is normally carried out by a professional hairdresser. Permanent colouration accounts for the largest section of the hair-dye market.

An important consumer demand exists for a convenient product, to be used at home, to restore to a dark colour hair which has become grey/white or has reddened. The latter occurs as a consequence of the exposure of black hair to sunlight. Black colours cannot be satisfactorily achieved with a direct dye and oxidation dyes suffer from the disadvantages referred to above.

Dark colouration of hair can, however, be achieved by the application of a solution comprising a dark coloured iron complex, which is formed by the reaction of an iron salt and an organic compound (which has the ability to react with iron ions to form a dark complex). The colour achieved is durable, resisting repeated washings (JP-A-60 209 513). Thus, a permanent dark colouration can be achieved more simply and conveniently than is possible with oxidation dyes. The uptake of the iron complex can be improved by pretreating the hair with a reagent such as L-cysteine, to "activate" the hair fibre to the uptake of a dark iron-polyhydroxyphenol complex which is subsequently added (JP-A-53 148 548). An alternative approach involves a pretreatment step with dilute alkali solution, followed by separate solutions of an iron (II) salt and of tannic acid. These are applied in succession to the hair, generating a dark colour in situ (JP-A-49 036 838).

Such formulations however lack consumer appeal since many consumers are reluctant to purchase and then use a hair-dye product per se. Furthermore, in many instances the product is inevitably dark in colour and therefore unattractive to purchase and to use.

DE-A-2028818 claims a process for colouring human hair which comprises firstly applying to the hair a pretreatment preparation and then treating the hair with an oxidation dye base and an oxidising mixture in which there is an oxidiser for the dye base that can form redox systems with the ions contained in the pretreatment preparation.

Chemical Abstract, Vol.94, 14, No. 109084X discloses a two component composition containing a conditioning composition which is applied daily and a colouring composition which is applied every three days.

It is an object of the present invention to overcome or at least mitigate the disadvantages of the prior art formulations.

It has been found that these disadvantages can be wholly or partially overcome by incorporating the reagents which combine together to form the dark colour into a two component, shampoo-based, hair care product.

Accordingly, the present invention provides a composition for darkening hair comprising:
(i) a first component, which comprises a shampoo detergent base and at least one metal salt (hereinafter referred to as the shampoo component); and
(ii) a second component, which comprises at least one organic compound (hereinafter referred to as the metal ligand) selected from the group comprising catechol, pyrogallol, gallic acid or an ester thereof or tannic acid or a mixture thereof, aromatic amines and precursors of melanin which, in use, changes colour as a consequence of the formation of a dark-coloured complex when mixed with the said metal ion, and a base suitable for use as a hair shampoo, conditioner, tonic, cream, styling gel or mousse with the proviso that neither hydrogen peroxide or urea are present.

The advantages offered by the invention are that the hair darkening can be effected personally, by the consumer, without recourse to professional aid, in a simple and convenient manner, during the course of normal hair washing and grooming operations, using a product which the consumer finds more attractive to purchase. It will be appreciated that the final dark colour is initially absent from either component, thereby adding to the consumer appeal. Whilst the initial application of a composition according to the inventions imparts an immediate darkening to the hair, the degree of darkening is progessively increased towards an end point, with successive applications of the composition. Accordingly, the consumer is able to control the degree of darkening effected. The darkening is of a semi-permanent nature so that once the desired darkening has been attained, the consumer is able to maintain that level and also compensate for natural hair growth, by less frequent application of the composition. In addition, and if the consumer should so desire, the colouring can be eventually reversed by repeated normal shampooing of the hair.

In a further aspect of the present invention there is provided a composition for darkening hair, consisting of:
(i) a first component, which comprises a shampoo detergent base and at least one metal salt; and
(ii) a second component, which comprises at least one organic compound selected from the group comprising catechol, pyrogallol, gallic acid or an ester thereof or tannic acid or a mixture thereof, aromatic amines and precursors of melanin which, in use, changes colour as a consequence of the formation of a dark-coloured complex when mixed with the said metal ion, and a base suitable for use as a hair shampoo, conditioner, tonic, cream, styling gel or mousse.

In the shampoo component of the composition of the invention, the metal of the metal salt is toxicologically acceptable and will form a dark coloured complex with an organic molecule. Examples of such metals include aluminium, gallium, indium, lead and the transition metals. Preferably the transition metal is selected from the group consisting of iron, chromium, copper, silver, manganese, cobalt, and zinc. Preferably the metal is iron, particularly iron in the oxidation state iron (II).

Whilst an iron (II) salt is particularly preferred for ease of formulation, it will be appreciated that on application, oxidation to iron (III) is a facile process. Suitable metal salts include halide, sulphate, nitrate, perchlorate, acetate, phosphate and citrate. The metal salt can also be present in the form of a mixed salt, for instance iron (II) ammonium sulphate.

Shampoo detergent bases suitable for use in hair care products comprise, as a major portion, an anionic surfactant. Suitable anionic surfactants for use in the compositions of the invention include alkyl sulphates, alkyl sulphonates, olefin sulphonates, alkyl benzene sulphonates, sulphosuccinate hemi-esters and fatty acid sarcosinates. Commonly used detergents of these kinds are the C₁₀-C₁₈ alkyl sulphates. These detergents are generally employed in the form of their sodium, potassium, magnesium, ammonium or mono-, di- or tri-ethanolamine salts. Examples of these detergents are sodium lauryl sulphate, magnesium lauryl sulphate, ammonium lauryl sulphate, and mono-, di- and tri-ethanolamine lauryl sulphates.

The shampoo detergent base may be present in the shampoo component of the composition of the invention from 0.1 to 80%, and preferably by 5 to 60%, by weight of the shampoo component.

The shampoo detergent base may also contain one or more other surfactants of an anionic, nonionic, amphoteric or cationic nature; to modify cleaning, conditioning and foaming properties. The latter property is preferably achieved by the inclusion in the shampoo detergent base of an amphoteric surfactant of the betaine type, such as (long chain alkyl)betaines or (long chain alkyl)amidoalkylbetaines. Particularly preferred is a cocamidoalkylbetaine such as cocamidopropylbetaine, being present in an amount of from 0.1 to 20% by weight of the shampoo component.

When the metal ion is iron (II), it is found that the presence of the amphoteric surfactant improves the storage stability of the shampoo component.

The shampoo component of the composition of the invention may be a clear, opaque or pearly liquid or a gel structure.

The shampoo component of the composition of the invention may contain minor amounts of antioxidants and sequestrants, to stabilise the metal ion in the shampoo detergent base, for instance, disodium edetate, sodium sulphite, thiourea, copper sulphate and vitamin E acetate.

The shampoo component of the composition of the invention may also include minor amounts of other ingredients which are commonly employed in shampoos, for example, viscosity modifiers, opacifier, perfume, colouring agent, preservative, protein and an agent for adjusting pH, the latter being preferably in the range 4 to 10, more preferably 4.5 to 8.

The shampoo component of the composition of the invention may be prepared by:
(a) optionally admixing a foam booster with the detergent base at a temperature of from 1 to 99°,
(b) mixing the shampoo base with the metal salt which may be optionally predissolved in water,
(c) and adjusting the pH of the composition to within the range 4 to 10, preferably from 4.5 to 8.

Further minor ingredients may be added at any convenient stage in the process, preferably before the final pH adjustment.

In the second component, examples of suitable metal ligands include ortho-substituted phenols, aromatic amines and precursors of melanin.

Examples of ortho-substituted phenols include salicaldehyde, salicyclic acid and ortho-dihydroxy benzene derivatives such as catechol, pyrocatechol, homocatechol, p-t-butyl catechol, pyrogallol, gallic acid or an ester thereof, tannic acid, 2,4,5-trihydroxytoluene, 1,2-dihydroxynaphthalene, 2,3-dihydroxynaphthalene, purporogallin, 3,4-dihydroxybenzaldehyde, 2,3-dihydroxybenzoic acid, 3,4-dihydroxybenzoic acid, caffeic acid, 1-(3,4-dihydroxyphenyl)-2-aminoethanol, 2,3-dihydroxynaphthalene-6-sulphonic acid, dopamine, 6-hydroxydopamine, 5,6-indol-2-carboxylic acid methyl ester 3,4-dihydroxycinnamic acid, 3,4-dihydroxyphenylacetic acid, 3,4-dihydroxyphenylethanol, 3,4-dihydroxyphenylethylene glycol, and phloroglucinol.

Examples of aromatic amines include phenyl or toluyl amines, optionally substituted with 1 to 3 groups selected from hydroxyl, alkoxy, amino or alkylamino.

Examples of precursors of melanin include tyrosine and DL-β-(3,4-dihydroxyphenyl)alanine and 5,6-dihydroxyindole.

Preferably the metal ligand is an ortho-dihydroxy benzene compound, particularly catechol, pyrogallol, gallic acid or an ester thereof or tannic acid or a mixture thereof.

Suitable esters of gallic acid include C₁₋₆ alkyl esters, for instance propyl.

A metal ligand can be used on its own or in combination with other metal ligands, depending on the exact shade of dark colour required.

The metal ligand, or if a mixture of ligands is used, then each individual one, is present in the second component of the composition of the invention in from 0.1 to 10%, preferably 0.1 to 5%, by weight of the composition.

The metal ligand is incorporated into any kind of formulation that is conventionally used after hair has been shampooed and rinsed, for instance, a hair shampoo, a hair conditioner, a hair tonic, a hair cream, a hair spray, a hair styling mousse or gel, to form the second component of the composition of the invention.

When the second component is a hair shampoo, it comprises a shampoo detergent base which is of the same general nature as that used for the first component, the shampoo component, but may or may not be identical with the first component.

When the second component is a hair conditioner, it comprises at least one surfactant which may be a cationic surfactant or an organofunctional silicone compound.

Preferred examples of cationic surfactants include quaternary ammonium compounds of the formula (I): 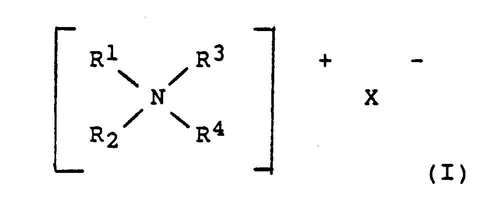
wherein:
X is an anion, preferably a halide (particularly chloride), methyl sulphate or ethanoate;
R¹ and R² are each alkyl or hydroxy substituted alkyl of 1 of 3 carbon atoms;
R³ is hydrogen, alkyl of 1 to 3 carbon atoms, benzyl, a polyethoxy or polypropoxy moiety containing up to 10 ethylene oxide units, alkoxypropyl or hydroxy substituted alkoxypropyl wherein the alkoxy moiety contains 12 to 20 carbon atoms, alkyl of 12 to 20 atoms, or alkylamidopropyl wherein the alkyl moiety contains from 12 to 20 carbon atoms; and
R⁴ is alkyl of 12 to 20 carbon atoms, a polyethoxy or a polypropoxy moiety containing up to 10 ethylene oxide units, alkyl acetate wherein the alkyl moiety contains 12 to 20 carbon atoms or alkylamido propyl wherein the alkyl moiety contains 12 to 20 carbon atoms;
quaternised guar gum derivatives; quaternised cellulose derivatives; polyhalide salts of polyionenes terminated with triethanolammonium groups; quaternised derivatives of gluconic acid; quaternary oleyl amine esters; synthetic polymers formed by the condensation of polyglycols with polyamines, polymers formed by combining vinyl pyrrolidone units with dimethylamine ethyl methacrylate units and homopolymers of dimethyl diallyl ammonium chloride.

Particularly useful quaternary ammonium compounds include:
Stearyldimethylbenzyl ammonium chloride,
Cetyltrimethyl ammonium bromide,
Distearyldimethyl ammonium chloride,
Di(hydrogenated tallow)dimethyl ammonium chloride,
Bis(2-hydroxyethyl)tallow ammonium ethanoate,
Stearamidopropyldimethyl(myristylacetate)ammonium chloride.

Preferred examples of organofunctional silicones are diaminofunctional silicones of general formula (II) 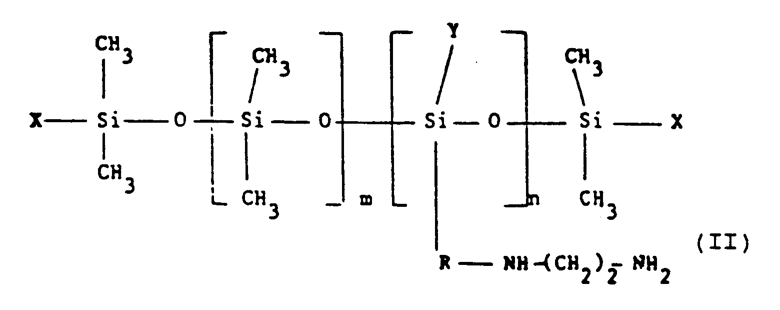
in which X and Y, which may be the same or different, are each C₁₋₆ alkyl or -OH, R is C₁₋₃ alkylene and m and n are each integers of from 1 to 100;
(β-hydroxy ammonium) functional silicones of general formula (III) 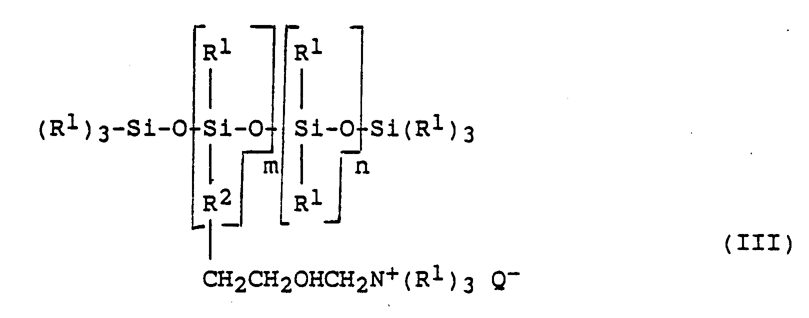
in which R¹ is C₁₋₈ alkyl or alkenyl, preferably methyl; R² is C₁₋₈ alkylene or C₁₋₈ alkylenoxy; Q⁻ is a halide ion, preferably chloride; and m and n are each integers from 1 to 100;
and amphoteric silicones.

Particularly preferred compounds of formula (II) are amodimethicone, wherein X and Y are -OH and R is -(CH₂)₃- and trimethylsilylamodimethicone, wherein X and Y are -CH₃ and R is -(CH₂)₃-.

Amodimethicone, in combination with polyoxyethylene(10)nonyl phenyl ether, is sold by Dow Corning as 'Dow Corning 929 emulsion', whilst in combination with polyoxyethylene(10)nonyl phenyl ether and tallow trimonium chloride, is sold by Dow Corning as 'Dow Corning 929 cationic emulsion'.

Trimethylsilylamodimethicone, in combination with octoxynol-40 and Isolaureth-6 is sold by Dow Corning as 'Dow Corning Q2-7224'.

Particularly preferred compounds of formula (III) are manufactured by Goldschmidt under the trademark Abil B9906.

Preferred examples of amphoteric organofunctional silicones are polysiloxane polyorganobetaine copolymers, a commercially available example being manufactured by Goldschmidt under the trade mark Abil B9950.

The total surfactant in the conditioner component may be present in that component in an amount of from 0.1 to 20% and preferably 0.1 to 10% by weight of the conditioner.

The conditioner component of the composition of the invention may also include minor amounts of other ingredients which are commonly employed in conditioners such as thickeners, suspending agents, sequestrants preservatives, perfumes, glycols. The conditioner base may optionally contain minor quantities of agents such as thiourea to stabilise the composition.

The conditioner may include a liquid vehicle, preferably water.

The conditioner component of the composition of the invention may be prepared by:
(a) optionally dissolving thickeners in a liquid vehicle, heating if necessary to between 30 and 90° to effect dissolution,
(b) admixing the surfactant with the liquid vehicle at ambient or slightly elevated temperature,
(c) admixing the metal ligand which may optionally be predispersed, suspended or dissolved in a suitable solvent, preferably a glycol, for example glycerin or propylene glycol, with the liquid vehicle.
(d) and adjusting the pH of the composition to within the range 3 to 10, preferably from 5 to 8.

Further toiletries additives may be added at any convenient stage in the process, preferably before final pH adjustment.

When the second component is a hair spray such as an aerosol hair spray, it comprises a resin material dissolved in a suitable solvent.

The hair-holding resin may be any of those which are conventionally used in aerosol hairspray formulations for holding hair in place.

Examples include copolymers of vinylpyrollidone/vinyl acetate, vinylacetate/crotonic acid and the butyl half ester of vinylmethyl ether/maleic anhydride.

Further examples include homopolymers of dimethylhydantoin/formaldehyde and vinylpyrrolidone; terpolymers such as vinylacetate/crotonic acid/vinyl neodecanoate terpolymers; and acrylic resins such as octylacrylamide/acrylate/butylamino-ethyl methyacrylate polymer, and acrylate/acrylamide copolymer.

The proportion of resin in the formulation is preferably between 0.5 and 10% by weight of the formulation.

The solvent system can be any of those conventionally used in resin hairspray formulations, but preferably comprises a C₁₋₆ alkanol, suitably ethanol. The proportion of solvent will vary according to the resin content of the formulation but is preferably from 20 to 90% by weight of the formulation. If desired, the formulation may also contain conventional plasticising materials such as a dimethicone or a dimethicone copolyol, for instance Dow Corning 190 or 193, manufactured by Dow Corning Corporation or a cyclomethicone, for instance Dow Corning 344 or 345, manufactured by Dow Corning Corporation. In addition the formulation will comprise a propellant material such as a mixture of fluorocarbons or hydrocarbons, and will be packed in a pressurised dispenser.

When the second component is a setting lotion or styling gel, it comprises a resin material dissolved in a suitable solvent. The resin may be any of those which are conventionally used in setting lotions or styling gels, for instance polyvinylpyrrolidone, copolymers of vinylpyrrolidone/vinyl acetate or crotonic acid/vinylacetate or alkylvinylethers or esters/maleic acid hemi-esters. The solvent is a hydro-alcoholic solution and, if desired a conventional plasticiser for the resin may also be included.

When the second component is a hair cream, it comprises a brilliantine or a water-in-oil or an oil-in-water emulsion, which are conventionally used in hair cream formulations. A water-in-oil emulsion is conventionally prepared using mixtures of calcium hydroxide, which can be used in the form of lime water, with fatty acids such as beeswax or paraffin wax; in combination with an oil such as mineral oil.

Preferably the second component comprises a base suitable for use as a hair conditioner.

The present invention also provides a multi-compartment device or kit for darkening human hair which comprises in a first compartment, a shampoo component (as hereinbefore defined) and in a second compartment, a composition comprising at least one metal ligand (as hereinbefore defined) and a base suitable for use as a hair conditioner, tonic, cream, styling gel or mousse with the proviso that neither hydrogen peroxide or urea are present.

The present invention further provides a method for treating hair comprising applying an effective amount of the two components of the composition of the invention to the hair, the first component being applied first, and then rinsed off and the second component being applied second and then either left on or rinsed off.

When the first component is rinsed off, the degree of dark colouration attained may be controlled by increasing the time interval between addition of the second component and subsequent removal by rising.

The composition of the invention will now be illustrated by way of the following Examples.

### Example 1

### Shampoo/conditioner

| A. Shampoo | |
|---|---|
| | % w/w |
| Sodium lauryl sulphate | 40.00 |
| Cocamidopropyl betaine | 10.00 |
| Iron (II) ammonium sulphate | 1.00 |
| Copper sulphate | 0.20 |
| Thiourea | 0.10 |
| Disodium edetate | 0.10 |
| Perfume | 0.50 |
| Sodium chloride | 9.50 |
| * Kathon C.G. (preservative) | 0.10 |
| Water | to 100.00% |

| B. Conditioner | |
|---|---|
| | % w/w |
| Dow Corning 929 cationic emulsion | 3.00 |
| ** Emcol CC9 | 0.3 |
| Hydroxypropylguar gum | 0.90 |
| Tannic acid | 0.50 |
| Catechol | 0.50 |
| Thiourea | 0.10 |
| Disodium edetate | 0.10 |
| Glycerin | 4.50 |
| Perfume | 0.50 |
| * Kathon C.G. | 0.10 |
| Deionised water | to 100.00% |
| Dow Corning 929 cationic emulsion is a combination of amodimethicone, tallowtrimonium chloride and polyoxyethylene(10)nonylphenyl ether, sold by Dow Corning. | |

| | |
|---|---|
| * Kathon C.G. is an aqueous solution of 5-chloro-2-methyl-4-isothiazolin-3-one, 2-methyl-4-isothiazolin-3-one, magnesium chloride and magnesium nitrate. | |
| ** Emcol CC9 is polyoxypropylene(9)methyldiethyl ammonium chloride. | |

### est Procedure

When the shampoo and conditioner were applied successively to the hair in the normal order, a colouring or darkening effect was seen. Successive applications increase the effect such that:
(a) When applied to hair which was originally 90% grey, an intense dark colouration was achieved after 7 applications
(b) When applied to hair which was originally 75% black/25% grey, the grey hairs are blended in with the background colour, making then indistinguishable from the background colour.

### Example 2

### Shampoo/conditioner

| A. Shampoo | |
|---|---|
| | % w/w |
| Magnesium lauryl sulphate | 40.00 |
| Cocamidopropyl betaine | 10.00 |
| * Kathon C.G. | 0.10 |
| Disodium edetate | 0.30 |
| Iron (II) ammonium sulphate | 1.00 |
| Ascorbic acid | 0.10 |
| Urea | 0.10 |
| Ammonia solution (0.28% solution) | 0.75 |
| Hexylene glycol | 2.30 |
| Perfume | q.s. |
| Deionized water | to 100.00% |

| B. Conditioner | |
|---|---|
| | % w/w |
| Hydroxypropylguar gum | 0.90 |
| Dow Corning 929 cationic emulsion | 3.00 |
| ** Emcol CC9 | 0.30 |
| * Kathon C.G. | 0.10 |
| Disodium edetate | 0.30 |
| Propyl gallate | 0.50 |
| Perfume | q.s. |
| Deionised water | to 100.00% |

| | |
|---|---|
| * Kathon C.G. is an aqueous solution of 5-chloro-2-methyl-4-isothiazolin-3-one, 2-methyl-4-isothiazolin-3-one, magnesium chloride and magnesium nitrate. | |
| ** Emcol CC9 is polyoxypropylene(9)methyldiethyl ammonium chloride. | |

### Example 3

### Shampoo/conditioner

| A. Shampoo | |
|---|---|
| | % w/w |
| Magnesium lauryl sulphate | 40.00 |
| Cocamidopropyl betaine | 5.00 |
| * Kathon C.G. | 0.10 |
| Disodium edetate | 0.30 |
| Iron (II) ammonium sulphate | 1.00 |
| Vitamin E acetate | 0.10 |
| Magnesium chloride | 0.5 |
| Perfume | q.s. |
| Deionized water | to 100.00% |

| B. Conditioner | |
|---|---|
| | % w/w |
| Ammonyx 4002 | 0.80 |
| Cetrimide | 0.70 |
| Hydroxypropylguar gum | 0.80 |
| Cetyl alcohol | 2.50 |
| Glyceryl monostearate | 0.80 |
| * Kathon C.G. | 0.10 |
| Tannic acid | 0.50 |
| Disodium edetate | 0.30 |
| Perfume | q.s |
| Deionized water | to 100.00% |
| Ammonyx 4002 is Stearyldimethylbenzyl ammonium chloride. | |
| Cetrimide is Cetyltrimethyl ammonium bromide. | |
| The shampoo component and hair conditioner are left on for 1 minute and 2 to 5 minutes, respectively, before being rinsed off. | |

| | |
|---|---|
| * Kathon C.G. is an aqueous solution of 5-chloro-2-methyl-4-isothiazolin-3-one, 2-methyl-4-isothiazolin-3-one, magnesium chloride and magnesium nitrate. | |

In Examples 4-7, further examples of the second component are given, to be used in combination with a first (shampoo)component, such as that described in Examples 1 to 3.

### Example 4

| Conditioner | |
|---|---|
| | % w/w |
| Ammonyx 4002 | 0.80 |
| Cetrimide | 0.70 |
| Natrosol 250 HHBR | 0.80 |
| Cetyl alcohol | 2.50 |
| Glyceryl monostearate | 0.80 |
| * Kathon C.G. | 0.10 |
| Propyl gallate | 0.50 |
| Glycerine | 4.50 |
| Disodium edetate | 0.30 |
| Perfume | q.s |
| Deionized water | to 100.00% |
| Natrosol 250 HHBR is Hydroxyethyl cellulose. | |

| | |
|---|---|
| * Kathon C.G. is an aqueous solution of 5-chloro-2-methyl-4-isothiazolin-3-one, 2-methyl-4-isothiazolin-3-one, magnesium chloride and magnesium nitrate. | |

### Example 5

| Hair Spray | |
|---|---|
| | % w/w |
| Octylacrylamide/Acrylates/butylamino Methacrylates Copolymer | 9.60 |
| 2-amino-2-methylpropan-1-ol | 0.34 |
| Dimethicone Copolyol | 0.09 |
| Glycerin | 0.20 |
| Ethoxylated fatty alcohol | 0.22 |
| Cyclomethicone pentamer | 0.20 |
| Propyl gallate | 0.50 |
| Ethanol | 88.15 |

### Example 6

| Styling Gel | |
|---|---|
| | % w/w |
| Carbopol | 0.80 |
| Triethanolamine | 0.40 |
| Polyvinylpyrrolidone | 1.50 |
| Polyethylene glycol | 0.75 |
| Propyl Gallate | 0.50 |
| Anti-Oxidants | q.s |
| Perfume | q.s |
| Water | to 100.00% |

### Example 7

| Shampoo | |
|---|---|
| Magnesium lauryl sulphate | 40.00 |
| Cocamidopropyl betaine | 5.00 |
| * Kathon C.G. | 0.10 |
| Disodium edetate | 0.30 |
| Propyl gallate | 0.50 |
| Hexylene glycol | 0.5 |
| Perfume | q.s. |
| Deionized water | to 100.00% |

| | |
|---|---|
| * Kathon C.G. is an aqueous solution of 5-chloro-2-methyl-4-isothiazolin-3-one, 2-methyl-4-isothiazolin-3-one, magnesium chloride and magnesium nitrate. | |

## Claims

1. A composition for darkening hair, consisting of:
(i) a first component, which comprises a shampoo detergent base and at least one metal salt; and
(ii) a second component, which comprises at least one organic compound selected from the group comprising catechol, pyrogallol, gallic acid or an ester thereof or tannic acid or a mixture thereof, aromatic amines and precursors of melanin which, in use, changes colour as a consequence of the formation of a dark-coloured complex when mixed with the said metal ion, and a base suitable for use as a hair shampoo, conditioner, tonic, cream, styling gel or mousse.

2. A composition for darkening hair, comprising:
(i) a first component, which comprises a shampoo detergent base and at least one metal salt; and
(ii) a second component, which comprises at least one organic compound selected from the group comprising catechol, pyrogallol, gallic acid or an ester thereof or tannic acid or a mixture thereof, aromatic amines and precursors of melanin which, in use, changes colour as a consequence of the formation of a dark-coloured complex when mixed with the said metal ion, and a base suitable for use as a hair shampoo, conditioner, tonic, cream, styling gel or mousse with the proviso that neither hydrogen peroxide or urea are present.

3. A composition as claimed in claim 1 or 2 in which the metal salt is an iron (II) salt.

4. A composition as claimed in claim 3 in which the metal salt is iron (II) ammonium sulphate.

5. A composition as claimed in any one of claims 1 to 4 in which the shampoo detergent base comprises, as a major portion, an anionic surfactant.

6. A composition as claimed in claim 5 in which the shampoo detergent base further contains an amphoteric surfactant.

7. A composition as claimed in claim 6 in which the amphoteric surfactant is a (long chain alkyl)betaine or a (long chain alkyl)amidoalkylbetaine.

8. A composition as claimed in any one of claims 1 to 7 in which, when the second component is a hair conditioner, said component comprises at least one surfactant which is a cationic surfactant or an organofunctional silicone compound.

9. A composition as claimed in claim 8 in which the cationic surfactant is selected from the group consisting of:
quartenary ammonium compounds of the formula (I): 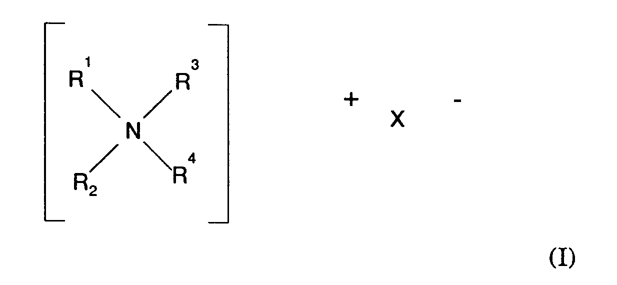 wherein:
X is an anion, preferably a halide (particularly chloride), methyl sulphate or ethanoate;
R¹ and R² are each alkyl or hydroxy substituted alkyl of 1 of 3 carbon atoms;
R³ is hydrogen, alkyl of 1 to 3 carbon atoms, benzyl, apolyethoxy or polypropoxy moiety containing up to 10 ethylene oxide units, alkoxypropyl or hydroxy substituted alkoxypropyl wherein the alkoxy moiety contains 12 to 20 carbon atoms, alkyl of 12 to 20 atoms, or alkylamidopropyl wherein the alkyl moiety contains from 12 to 20 carbon atoms; and
R₄ is alkyl of 12 to 20 carbon atoms, a polyethoxy or a polypropoxy moiety containing up to 10 ethylene oxide units, alkyl acetate wherein the alkyl moiety contains 12 to 20 carbon atoms or alkylamido propyl wherein the alkyl moiety contains 12 to 20 carbon atoms;
quaternised guar gum derivatives; quaternised cellulose derivatives;
polyhalide salts of polyionenes terminated with triethanolammonium groups;
quaternised derivatives of gluconic acid; quaternary oleyl amine esters;
synthetic polymers formed by the condensation of polyglycols with polyamines, polymers formed by combining vinyl pyrrolidone units with dimethylamine ethyl methacrylate units and homopolymers of dimethyl diallyl ammonium chloride.

10. A composition as claimed in claim 8 in which the organo functional silicone is a diamino functional silicone of general formula (II) 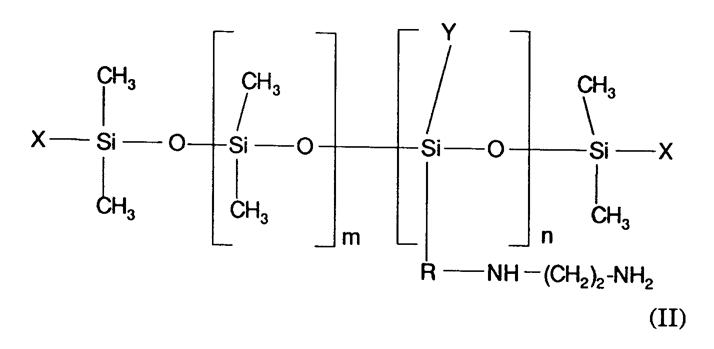 in which X and Y, which may be the same or different, are each C₁₋₆ alkyl or -OH, R is C₁₋₃ alkylene and m and n are each integers of from 1 to 100;
or a (β-hydroxy ammonium) functional silicone of general formula (III) 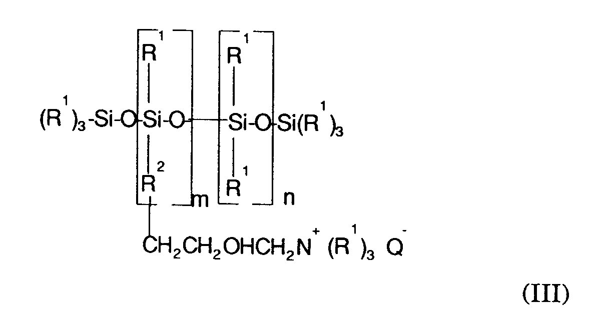 in which R¹ is C₁₋₈ alkyl or alkenyl, preferably methyl; R² is C₁₋₈ alkylene or C₁₋₈ alkylenoxy; Q- is a halide ion, preferably chloride; and m and n are each integers from 1 to 100;
or an amphoteric silicone.

11. A composition as claimed in claim 10 in which the compound of formula (II) is:
amodimethicone, wherein X and Y are -OH and R is -(CH₂)₃-; or
trimethylsilyl-amodimethicone, wherein X and Y are -CH₃ and R is -(CH₂)₃-.

12. A multi-compartment device or kit for darkening human hair which comprises, in a first compartment, a shampoo component as defined in any one of claims 1 to 7, and in a second compartment, an organic compound as defined in claim 1 or 2, and a base suitable for use as a hair shampoo, conditioner, tonic, cream, styling gel or mousse with the proviso that neither hydrogen peroxide or urea are present.

13. A method for treating hair comprising applying an effective amount of the two components of a composition as defined in any one of claims 1 to 11, the first component being applied first, and then rinsed off and the second component being applied second and then either left on or rinsed off.

## Patentansprüche

1. Zusammensetzung zum Dunkelfärben von Haar, bestehend aus:
(i) einer 1. Komponente, die einen Haarwaschgrundstoff und mindestens ein Metallsalz umfaßt, und
(ii) einer 2. Komponente, umfassend mindestens eine organische Verbindung ausgewählt aus der Gruppe umfassend Brenzcatechin, Pyrogallol, Gallussäure oder deren Ester oder Gerbsäure oder deren Gemisch, aromatischen Aminen und Vorstufen von Melanin, die beim Anwenden die Farbe ändert als Folge der Bildung eines dunkel-gefärbten Komplexes beim Vermischen mit dem Metallion, und einen Grundstoff zur Verwendung als Haar-Shampoo, -Festiger, -Tonikum, -Creme, -Styling Gel oder -Schaum.

2. Zusammensetzung zum Dunkelfärben von Haar, umfassend:
(i) eine 1. Komponente, die einen Haarwaschgrundstoff und mindestens ein Metallsalz umfaßt, und
(ii) eine 2. Komponente, die mindestens eine organische Verbindung ausgewählt aus der Gruppe umfassend Brenzcatechin, Pyrogallol, Gallussäure oder deren Ester oder Gerbsäure oder deren Gemisch, aromatischen Aminen und Vorstufen von Melanin, die beim Anwenden die Farbe ändert als Folge der Bildung eines dunkel-gefärbten Komplexes beim Vermischen mit dem Metallion, und einen Grundstoff zur Verwendung als Haar-Shampoo, -Festiger, -Tonikum, -Creme, -Styling Gel oder -Schaum, mit der Maßgabe, daß weder Wasserstoffperoxid noch Harnstoff vorliegen.

3. Zusammmensetzung nach Anspruch 1 oder 2, wobei das Metallsalz ein Eisen(II)-Salz ist.

4. Zusammensetzung nach Anspruch 3, wobei das Metallsalz Eisen(II)ammoniumsulfat ist.

5. Zusammensetzung nach einem der Ansprüche 1 bis 4, wobei der Haarwaschgrundstoff als Hauptbestandteil ein anionisches Tensid umfaßt.

6. Zusammensetzung nach Anspruch 5, wobei der Haarwaschgrundstoff außerdem ein amphoteres Tensid enthält.

7. Zusammensetzung nach Anspruch 6, wobei das amphotere Tensid ein (langkettiges Alkyl-)Betain oder ein (langkettiges Alkyl-)Amidoalkylbetain ist.

8. Zusammensetzung nach einem der Ansprüche 1 bis 7, wobei, falls die 2. Komponente ein Haarfestiger ist, diese mindestens ein Tensid umfaßt, das ein kationisches Tensid oder eine organofunktionelle Siliconverbindung ist.

9. Zusammensetzung nach Anspruch 8, wobei das kationische Tensid ausgewählt ist aus der Gruppe der quartären Ammoniumverbindungen der Formel (I): 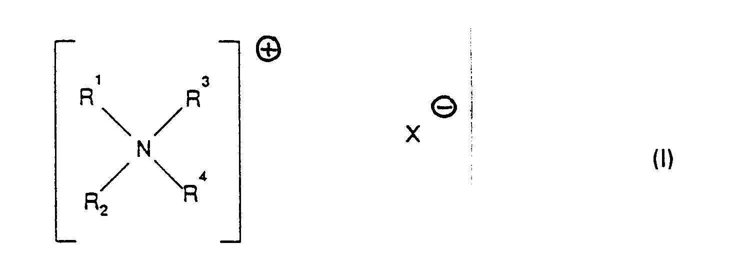 wobei X ein Anion, vorzugsweise ein Halogenid- (insbesondere ein Chlorid-), Methylsulfat- oder Ethoxid-anion ist,
die Reste R¹ und R² jeweils C₁₋₃-Alkyl- oder Hydroxy-C₁₋₃-Alkylgruppen sind,
der Rest R³ ein Wasserstoffatom, eine C₁₋₃-Alkylgruppe, eine Benzyl-, Polyethoxy- oder Polypropoxygruppe mit bis zu 10 Ethylenoxideinheiten, eine C₁₂₋₂₀-Alkoxypropyl- oder Hydroxy-C₁₂₋₂₀-Alkoxypropylgruppe ist, eine C₁₂₋₂₀-Alkylgruppe oder eine C₁₂₋₂₀-Alkyl-amidopropylgruppe enthält,
und der Rest R⁴ eine C₁₂₋₂₀-Alkylgruppe, eine Polyethoxy- oder Polypropoxykomponente mit bis zu 10 Ethylenoxideinheiten, C₁₂₋₂₀-Alkylacetat oder eine C₁₂₋₂₀-Alkyl-amidopropylgruppe ist,
oder aus quarternisierten Guaran-Derivaten, quarternisierten Cellulose-Derivaten, Polyhalogenidsalzen von polykationischen Verbindungen mit Triethanolammoniumendgruppen, quarternisierten Derivaten der Gluconsäure, quartären Oleylaminestern, durch Kondensation von Polyglycolen mit Polyaminen hergestellten synthetischen Polymeren, durch Copolymerisation von Vinylpyrrolidon- und Dimethylaminethylmethacrylat-Grundbausteinen hergestellten Copolymerisaten und Homopolymerisaten von Dimethyldiallylammoniumchlorid.

10. Zusammensetzung nach Anspruch 8, wobei das organofunktionelle Silicon ein Diamino-funktionelles Silicon entweder der allgemeinen Formel (II) 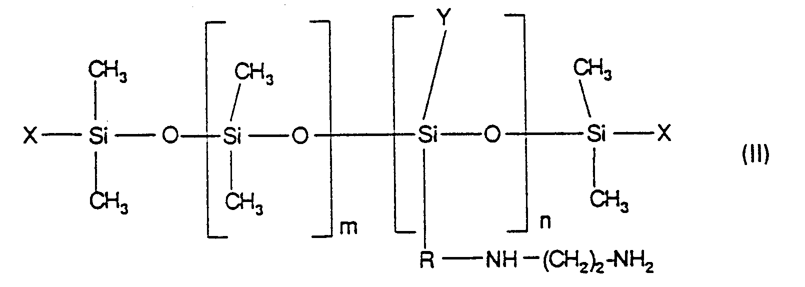 in der X und Y gleich oder verschieden und C₁₋₆-Alkyl- oder Hydroxyl-Gruppen sind, der Rest R eine C₁₋₃-Alkylengruppe ist und m und n jeweils ganze Zahlen mit einem Wert von 1 bis 100 sind,
oder ein (β-Hydroxyammonium)-funktionelles Silicon der allgemeinen Formel (III) 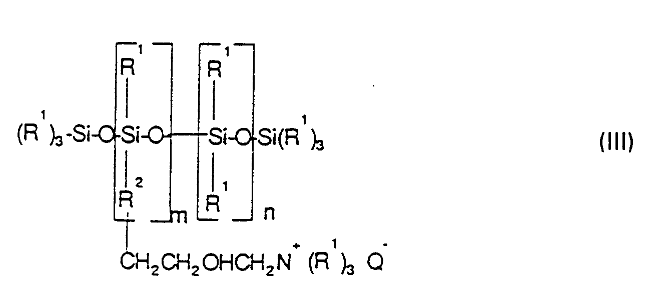 in der der Rest R¹ eine C₁₋₈-Alkylgruppe, vorzugsweise eine Methylgruppe, oder eine Alkenylgruppe ist, der Rest R² eine C₁₋₈-Alkylen- oder C₁₋₈-Alkylenoxygruppe ist, Q ein Halogenidion, vorzugsweise ein Chloridion ist, und m und n jeweils ganze Zahlen mit einem Wert von 1 bis 100 sind,
oder ein amphoteres Silicon ist.

11. Zusammensetzung nach Anspruch 10, in der die Verbindung der Formel (II) Amodimethicon ist, wobei die Reste X und Y entweder Hydroxy-Gruppen sind und der Rest R ein -(CH₂)₃-Rest ist, oder Trimethylsilylamodimethicon ist, wobei die Reste X und Y CH₃-Reste sind und der Rest R ein -(CH₂)₃-Rest ist.

12. Eine mehrere Behältnisse umfassende Einrichtung oder Ausrüstung zum Dunkelfärben menschlicher Haare, umfassend:
in einem ersten Behältnis eine Shampookomponente nach einem der Ansprüche 1 bis 7 und
in einem zweiten Behältnis eine organische Verbindung nach Anspruch 1 oder 2 und einen Grundstoff zur Verwendung als Haarshampoo, -Festiger, -Tonikum, -Creme, -Styling Gel oder -Schaum, mit der Maßgabe, daß weder Wasserstoffperoxid noch Harnstoff liegen.

13. Verfahren zur Haarbehandlung, umfassend das Auftragen einer wirksamen Menge der beiden Komponenten einer Zusammensetzung nach einem der Ansprüche 1 bis 11, wobei zuerst die erste Komponente aufgetragen und dann ausgespült wird und danach die zweite Komponente aufgetragen wird und entweder auf dem Haar belassen oder ausgespült wird.

## Revendications

1. Composition pour rendre les cheveux plus foncés, consistant en :
(i) un premier composant, qui comprend une base de détergent pour shampooing et au moins un sel métallique; et
(ii) un second composant, qui comprend au moins un composé organique choisi dans le groupe comprenant le catéchol, le pyrrogallol, l'acide gallique ou un ester de ceux-ci, ou l'acide tannique ou un mélange de ceux-ci, des amines aromatiques et des précurseurs de mélamine, qui en cours d'utilisation, change de couleur à la suite de la formation d'un complexe de couleur foncée lorsqu'il est mélangé à cet ion métallique, et une base convenable utile en tant que shampooing capillaire, agent de conditionnement, tonique, crème, gel ou mousse de modelage.

2. Composition pour rendre les cheveux plus foncés, consistant en :
(i) un premier composant, qui comprend une base de détergent pour shampooing et au moins un sel métallique; et
(ii) un second composant, qui comprend au moins un composé organique choisi dans le groupe comprenant le catéchol, le pyrrogallol, l'acide gallique ou un ester de ceux-ci, ou l'acide tannique ou un mélange de ceux-ci, des amines aromatiques et des précurseurs de mélamine, qui en cours d'utilisation, change de couleur à la suite de la formation d'un complexe de couleur foncée lorsqu'il est mélangé à cet ion métallique, et une base convenable utile en tant que shampooing capillaire, conditionneur, tonique, crème, gel ou mousse de modelage, à condition qu'il n'y ait ni peroxyde d'hydrogène ni urée.

3. Composition suivant les revendications 1 ou 2, dans laquelle le sel métallique est un sel de fer (II).

4. Composition suivant la revendication 3, dans laquelle le sel métallique est le sulfate d'ammonium et de fer (II).

5. Composition suivant l'une quelconque des revendications 1 à 4, dans laquelle la base détergente de shampooing comprend un tensioactif anionique en tant que composant principal.

6. Composition suivant la revendication 5, dans laquelle la base de détergent pour shampooing contient de plus un tensioactif amphotère.

7. Composition suivant la revendication 6, dans laquelle le tensioactif amphotère est une alkyl-(à longue chaîne)-bétaïne ou une alkyl-(à longue chaîne)-amidoalkylbétaïne.

8. Composition suivant l'une quelconque des revendications 1 à 7, dans laquelle, lorsque le second composant est un agent de conditionnement capillaire, ce composant comprend au moins un tensioactif qui est un tensioactif cationique ou un composé silicone organofonctionnel.

9. Composition suivant la revendication 8, dans laquelle le tensioactif cationique est choisi dans le groupe consistant en :
composés ammonium quaternaire de formule (I) : 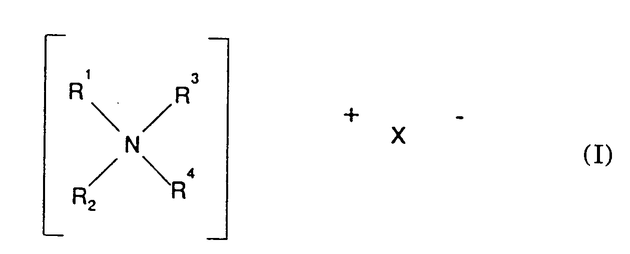 dans laquelle :
X est un anion, de préférence un halogénure (en particulier un chlorure), un méthyl sulfate ou un éthanoate;
R¹ et R² sont chacun un groupe alkyle ou alkyle substitué par un radical hydroxy ayant 1 à 3 atomes de carbone;
R³ est un atome d'hydrogène, un groupe alkyle de 1 à 3 atomes de carbone, benzyle, une partie polyéthoxy ou polypropoxy contenant jsuqu'à 10 unités oxyde d'éthylène, alcoxypropyle ou alcoxypropyle substitué par un radical hydroxy dans lesquels le groupe alcoxy contient 12 à 20 atomes de carbone, alkyle de 12 à 20 atomes de carbone ou alkylamidopropyle dans lequel la partie alkyle contient 12 à 20 atomes de carbone; et
R⁴ est un groupe alkyle de 12 à 20 atomes de carbone, une partie polyéthoxy ou polypropoxy contenant jusqu'à 10 unités oxyde d'éthylène, acétate d'alkyle dans lequel la partie alkyle contient 12 à 20 atomes de carbone, ou alkylamidopropyle dans lequel la partie alkyle contient 12 à 20 atomes de carbone;
dérivés de gomme guar quaternisés; dérivés cellulosiques quaternisés, sels polyhalogénures de polyionènes à terminaisons triéthanol-ammonium; dérivés quaternisés d'acide gluconique; oléyl amine esters quaternaires; polymères synthétiques formés par la condensation de polyglycols avec des polyamines, polymères formés par combinaison d'unités vinyl pyrrolidone avec des unités de méthacrylate de diméthylamine éthyle et homopolymères de chlorure de diméthyl diallyl ammonium.

10. Composition suivant la revendication 8, dans laquelle le silicone organofonctionnel est
un silicone à fonction diamino de formule générale (II) : 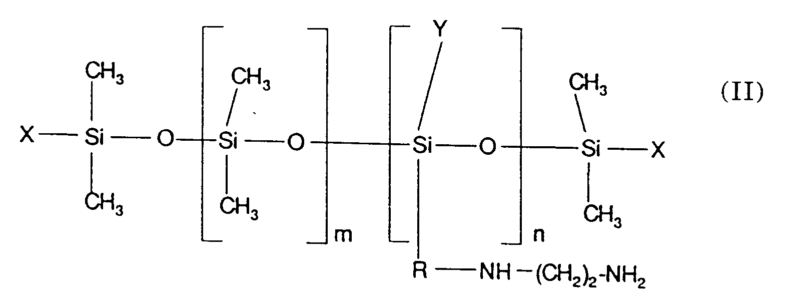 dans laquelle X et Y, qui peuvent être identiques ou différents, sont chacun un groupe alkyle en C₁₋₆ ou OH, R est un groupe alkylène en C₁₋₃ et m et n sont chacun des entiers de 1 à 100;
un silicone à fonction β-hydroxy-ammonium de formule générale (III) 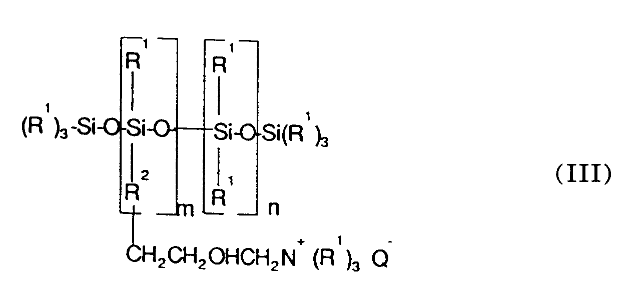 dans laquelle R¹ est un groupe alkyle ou alcényle en C₁₋₈, de préférence un groupe méthyle; R² est un groupe alkylène en C₁₋₈ ou alkylèneoxy en C₁₋₈; Q⁻ est un ion halogénure, de préférence un chlorure; et m et n sont chacun des entiers de 1 à 100;
ou un silicone amphotère.

11. Composition suivant la revendication 10, dans laquelle le composé de formule (II) est :
l'amodiméthicone, où X et Y sont -OH et R est (CH₂)₃-; ou
le triméthylsilyl-amodiméthicone, où X et Y sont -CH₃ et R est -(CH₂)₃-.

12. Dispositif ou trousse multi-compartiment pour rendre plus foncés les cheveux humains, qui comprend, dans un premier compartiment, un composant shampooing, tel que défini dans l'une quelconque des revendications 1 à 7 et dans un second compartiment, un composé organique tel que défini dans les revendications 1 ou 2, et une base convenable utile en tant que shampooing capillaire, agent de conditionnement, tonique, crème, gel ou mousse de modelage, à condition qu'il n'y ait ni peroxyde d'hydrogène, ni urée.

13. Procédé pour traiter des cheveux comprenant l'application d'une quantité efficace des deux composants d'une composition telle que définie dans l'une quelconque des revendications 1 à 11, le premier composant étant appliqué en premier lieu, et ensuite éliminé par rinçage et le second composant étant appliqué en second lieu et ensuite, soit laissé, soit éliminé par rinçage.
